# EUROPEAN PATENT APPLICATION

(11) **EP 1 880 696 A2**
(43) Date of publication of application: **23.01.2008**
(21) Application number: 07012289.0
(22) Date of filing: 22.06.2007
(51) Int. Cl.: A61F 5/01, A43B 7/26

(54) **Toe separator for sports shoes**

(30) Priority: 05.07.2006 EE 200600023
(71) Applicant: Zitin, Vladlen, 10124 Tallinn (EE)
(72) Inventor: Zitin, Vladlen, 10124 Tallinn (EE)
(74) Representative: Nelsas, Tónu

(57) **Abstract**

Athletic positioning force unifying separator for toes comprises a case made from bending material, comprising reinforcements of predetermined stiffness, depending the aim of the product, or does not include reinforcements. Separator has clearances of suitable form, so that the toe is surrounded by the partition walls, and suitable outer form for a footwear. In the manufacture of the case can be used pattern casting (synthetics, rubber etc.)

## Description

### Field of the invention

A toe separator used for sporting purposes (flexible, semi-flexible, semi-rigid). The separator is designed for the optimization of toe exertion and the integration of dynamic loads, ensuring smooth operation in the front part of the foot.

### Background of the invention

In many sports, toes and their joint functioning dictate the effectiveness of sporting results. Individually, the result of each toes' unsatisfactory functionality is dynamic overload, active and passive traumas, chronic injuries in football, hockey, track and field, skiing, etc.).

The footwear used in the above mentioned sports are not designed for increasing the properties of functionality, as they are in ballet (ballet slippers).

US 5 076 263 discloses the toe stretcher, which is held between the toes in order to keep the toes apart from one another and to massage the space between the toes. The toe extender contains extension posts made of elastic material, where each post is in the shape of the space between the toes. The posts are connected to each other by a frame, which in the preferred version is made of flexible foam material.

Attempts by people to optimize the functionality of toes can be illustrated by the following examples: use of fine-tuned leg supporters, finger binding used by boxers.

The purpose of the provided examples is the optimization of effort and prevention of traumas. The placement into use of the separator as a new product in sports may ensure the resolution of similar tasks.

### Summary of the invention

The separator is composed of a case prepared from flexible materials, which contains the previously described stiff reinforcements, depending on the products intended use, or which does not contain reinforced parts. The separator has properly shaped spaces for the areas between the toes, so that the toe is surrounded by a divider, and the proper external shape for the footwear. Mouldings (synthetic, rubber, etc.) can be used for the preparation of the shell.

The use of the separator in football ensures the accuracy of kicks and increases the power of kicks as well as the speed at which the ball flies. The inaccuracy of kicks is a testament to the inefficient cooperation between toes because in terms of their nature toes are not designed for kicking.

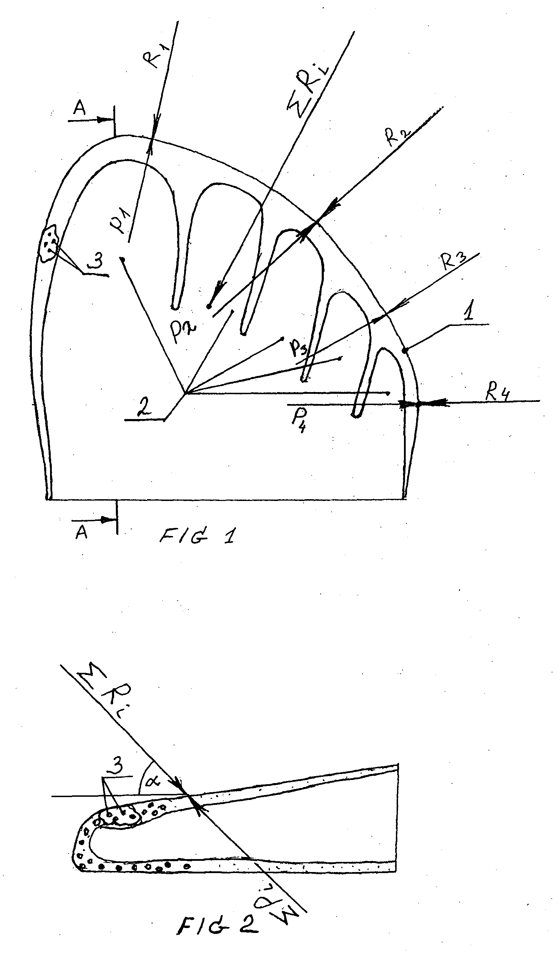

The purpose of the invention is to apply the working principle behind ball bearings to the toes. Toes attached to the separator convey the force of the kick through the separator, but the kick response is also accepted through separator 1 (see figure 1).

### Brief description of the drawings

figure 1 is an overhead cross-section view of the invented separator together with the front part of the foot,
figure 2 is a section A-A of the figure 1,
figure 3 is an overhead cross-section view of one of the alternative uses for the separator together with the front part of the foot,
figure 4 is a section A-A of the figure 3.

### Detailed description of the invention

The separator is composed of a shell made from flexible material, which contains the previously described stiff reinforcements, depending on the products intended use, or which does not contain reinforced parts.

The separator has properly shaped spaces for the areas in between the toes, so that the toe is surrounded by a divider, and the proper external shape for the footwear. Mouldings (synthetic, rubber, etc.) can be used during the preparation of the shell.

The toe separator illustrated in figure 1 contains a monolithic or a connected monolithic case 1, which is prepared from elastic material in the shape of the front of the foot, containing toes 2 identically shaped cavities for fixing the position of the toes. Case 1 contains elastic reinforced element 3, which ends in symmetrical or asymmetrical shaped rings. At the moment of impact with the ball various areas of the front of the foot are subjected to dynamic forces R₁, R₂, R₃, R₄, the foot, as a whole, is loaded with an integrated dynamic load of ΣRᵢ. At the same moment the force of the muscles of the foot develops a dynamic load in the opposite direction with a force of P₁, P₂, P₃, P₄, with the foot as a whole creating a force of ΣPᵢ. The case of the separator has ventilation openings (not shown on the drawings).

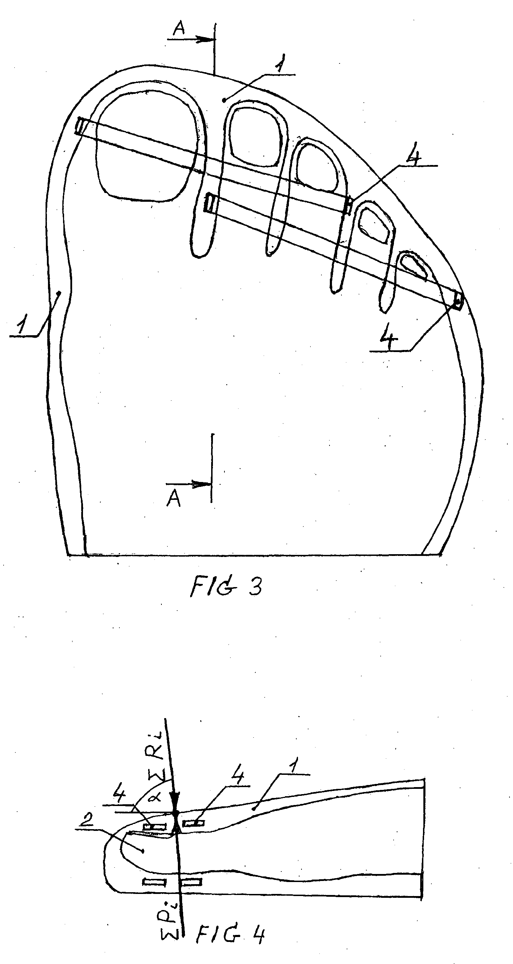

Figure 2 shows the dynamic load effecting the foot and the force created by the foot and the possible angles α, between the foot's longitudinal axis, where α may be between 0 and at least 90°.

Figure 3 shows one of the alternative uses for the separator together with a cross-section overhead view of the front part of the foot. Used as reinforced elements are local light metals, metal alloys, polymers, carbon and ceramic polymer based materials with multiple cross-sections, with a preference towards strip 4 shaped elements with the goal of increasing the rigidity and elasticity of the shell. There may be more than 1 of strip 4.

Figure 4 shows the 4 locations for the strips in terms of the toes.

The case of the separator may be composed of the same type of elastic material throughout the entire cross-section or in an alternative application the exterior part may be covered with a higher elasticity material than then internal layer, which comes in contact with the toes.

## Claims

1. Sporty, positionable, strength uniting toe separator, **characterized in that** it contains a monolithic or connected monolithic case (1) which is manufactured from elastic materials based on the shape of the front of the foot, containing identically shaped cavities in the shape of the toes for fixing the position of the toes, whereas the case contains elastic reinforced elements (3) which end in symmetrically or asymmetrically shaped rings; reinforced elements are local light metals, metal alloys, polymers, carbon and ceramic polymer based materials with multiple cross-sections, preferably in the shape of a plate, with the goal of increasing the case's rigidity and elasticity.

2. Sporty, positionable, strength uniting toe separator according to the claim 1, **characterized in that** the shells exterior part is covered with a higher elasticity material than the interior layer, which comes in contact with the toes.

3. Sporty, positionable, strength uniting toe separator according to the claim 1, **characterized in that** the case has ventilation openings.

4. Sporty, positionable, strength uniting toe separator according to the claim 1, **characterized in that** the separator is prepared from natural or artificial materials, which include the following: threads, fabric, wax, polymers, caoutchouc, rubber, latex and their combinations.
